# EUROPEAN PATENT SPECIFICATION

(11) **EP 1 688 492 B1**
(45) Date of publication and mention of the grant of the patent: **19.08.2009**
(21) Application number: 04799655.8
(22) Date of filing: 05.11.2004
(51) Int. Cl.: C12Q 1/68

(54) **METHOD AND KIT FOR ESTIMATING SIDE EFFECT BY PACLITAXEL THERAPY**
VERFAHREN UND KIT ZUR ABSCHÄTZUNG DER NEBENWIRKUNG DURCH EINE PACLITAXEL-THERAPIE
PROCEDE ET TROUSSE POUR L'ESTIMATION D'EFFET SECONDAIRE DE LA THERAPIE PAR PACLITAXEL

(30) Priority: 05.11.2003 JP 2003375369
(43) Date of publication of application: 09.08.2006
(73) Proprietor: JAPANESE FOUNDATION FOR CANCER RESEARCH, Tokyo 135-8550 (JP)
(72) Inventor: MIKI, Yoshio, c/o Japanese Fdt Cancer Research, Tokyo 135-8550 (JP); MATSUURA, Masaaki, c/oJapanese Fdt Cancer Research, Tokyo 135-8550 (JP); ISOMURA, Minoru, c/o Japanese Fdt Cancer Research, Tokyo 135-8550 (JP); MIYATA, Satoshi, c/o Japanese Fdt Cancer Research, Tokyo 135-8550 (JP); YOSHIMOTO, Masataka, Japanese FDt.Cancer Research, Tokyo 135-8550 (JP); NODA, Tetsuo, Japanese Fdt. for Cancer Research, Tokyo 135-8550 (JP)
(74) Representative: Bublak, Wolfgang
(86) International application number: PCT/JP2004/016805
(87) International publication number: WO 2005/045029

(56) References cited:
- WO-A2-02/099099
- WO-A2-03/045227
- JP-A- 2003 093 068
- US-B1- 6 528 260
- ISOMURA M ET AL: "Adverse effects of paclitaxel can be precisely predicted by combinations of polymorphisms on two genomic loci." AMERICAN JOURNAL OF HUMAN GENETICS, vol. 73, no. 5, November 2003 (2003-11), page 174, XP009074739 & 53RD ANNUAL MEETING OF THE AMERICAN SOCIETY OF HUMAN GENETICS; LOS ANGELES, CA, USA; NOVEMBER 04-08, 2003 ISSN: 0002-9297
- BAHADUR, N. ET AL.: 'CYP2C8 polymorphisms in Caucasians and their relationship with paclitaxel 6alpha-hydroxylase activity in human liver microsomes' BIOCHEM PHARMACOL vol. 64, no. 11, 2002, pages 1579 - 1589, XP002983729
- SOYAMA, A. ET AL.: 'Non-synonymous single nucleotide alterations found in the CYP2C8 gene result in reduced in vitro paclitaxel metabolism' BIOL PHARM BULL vol. 24, no. 12, 2001, pages 1427 - 1430, XP001121633
- DATABASE //SNP.IMS.U-TOKYO.AC.JP/ [Online] XP002983730 Database accession no. IMS-JST111898

### SEQUENCE LISTING

<110> Cancer Institute
<120> Method and Kit for Prediction of Adverse Effect of Paclitaxel
<130> PGK-9001WO
<150> JP 2003-375369
   <151> 2003-11-05
<160> 20
<170> Patent In version 3.1
<210> 1
   <211> 21
   <212> DNA
   <213> homo sapiens
<400> 1
   cagagcaagg rcaactgttt c 21
<210> 2
   <211> 21
   <212> DNA
   <213> homo sapiens
<400> 2
   tacttttacc ytaaatatga g 21
<210> 3
   <211> 21
   <212> DNA
   <213> homo sapiens
<400> 3
   gagatcagta raaacagtat g 21
<210> 4
   <211> 21
   <212> DNA
   <213> homo sapiens
<400> 4
   gaaatttcca wagtgctggt t 21
<210> 5
   <211> 21
   <212> DNA
   <213> homo sapiens
<400> 5
   attgctattt rtccatgatc a 21
<210> 6
   <211> 21
   <212> DNA
   <213> homo sapiens
<400> 6
   ggagtcgtgt rcgtgccttg g 21
<210> 7
   <211> 21
   <212> DNA
   <213> homo sapiens
<400> 7
   gactgacaca kaattattat t 21
<210> 8
   <211> 21
   <212> DNA
   <213> homo sapiens
<400> 8
   aactggctgt ygtgcagtct c 21
<210> 9
   <211> 21
   <212> DNA
   <213> homo sapiens
<400> 9
   aggaaggcaa yctgtttttt t 21
<210> 10
   <211> 21
   <212> DNA
   <213> homo sapiens
<400> 10
   gggtacatct yagctatgcc a 21
<210> 11
   <211> 121
   <212> DNA
   <213> homo sapiens
<400> 11
   aaaaagaaag gtcaaggcag gagcctcagc tcaggagaag aaacaaggag cagagcaagg 60
   rcaactgttt ctcaaggaat aaaattattg ctctaaagag agaaagtgaa cttattttat 120
   c 121
<210> 12
   <211> 121
   <212> DNA
   <213> homo sapiens
<400> 12
   caaattcccc atgtgtccaa aaaaaatcag catggatgaa ataaacacat tacttttacc 60
   ytaaatatga gttgagcatt acaggctagc taaacaatgt catttcgcat gtggttattc 120
   a 121
<210> 13
   <211> 121
   <212> DNA
   <213> homo sapiens
<400> 13
   ttgatgacac aatttaaaat gacatctttg tacaatggag gaggatgaca gagatcagta 60
   raaacagtat ggcagtagca aaataagtaa agcactgatg aagtgtctgg atttcagcaa 120
   a 121
<210> 14
   <211> 121
   <212> DNA
   <213> homo sapiens
<400> 14
   ctcatcccca aggtaagctt gtttctctta cactatattt ctgtacttct gaaatttcca 60
   wagtgctggt ttggttccaa ccctctaaca acacaagatg agagaagtgc aaaactcata 120
   c 121
<210> 15
   <211> 121
   <212> DNA
   <213> homo sapiens
<400> 15
   tttttggaat tagttggaat ttacatggca cctcctctgg ggctggtaga attgctattt 60
   rtccatgatc aagagcacca ctcttaacac ccatgtgctc caccctcaca atacaccatc 120
   a 121
<210> 16
   <211> 121
   <212> DNA
   <213> homo sapiens
<400> 16
   tttgaaactt ggcggctagg ggtgtgggct tgaggtggcc ggtttgttag ggagtcgtgt 60
   rcgtgccttg gtcgcttctg tagctccgag ggcaggttgc ggaagaaagc ccaggcggtc 120
   t 121
<210> 17
   <211> 121
   <212> DNA
   <213> homo sapiens
<400> 17
   taaatgtctt ccgaaaggtg attattcatg gtcttgggtt gaatatagtg gactgacaca 60
   kaattattat tattattata tgcctaagct tctttgttag ctgtttttca agtttatggc 120
   t 121
<210> 18
   <211> 121
   <212> DNA
   <213> homo sapiens
<400> 18
   cccaccctta ataattccca cttcaaaata tccaaaaacc acactcacat aactggctgt 60
   ygtgcagtct cttccacata tggagtgaaa ctgggaagca cagcgggtac agctatcagt 120
   g 121
<210> 19
   <211> 121
   <212> DNA
   <213> homo sapiens
<400> 19
   tcttcaagac aaccagataa attaatcaat attttgtgtt gtttgaaagc aggaaggcaa 60
   yctgtttttt taataacaaa aagcttcaaa catataaaag gtcattaaac aatttaccaa 120
   t 121
<210> 20
   <211> 121
   <212> DNA
   <213> homo sapiens
<400> 20
   aggccatgaa agaagctgca tagctggtct ttaaaaaaaa aaggtacctt gggtacatct 60
   yagctatgcc aacaactccc tccagtggtt aattttgaaa atgcacctgt aagacagagc 120
   a 121

## Description

### TECHNICAL FIELD

The present invention relates to a method for predicting the risk of the occurrence of granulocytopenia, one of the adverse side effects of paclitaxel therapy, by identifying genetic polymorphisms, and a diagnostic kit for carrying out that method.

### BACKGROUND ART

Paclitaxel is an alkaloid of a diterpene derivative that is also known by generic names such as Taxol (registered trademark). It is an antitumor drug that induces stabilization and excessive formation of microtubules by promoting microtubule protein polymerization, and inhibits mitosis by impairing the function of spindle bodies (Manfredi, J.J. and Horwitz, S.B.: Taxol: an antimitotic agent with a new mechanism of action, Pharmac. Ther., 25: 83-125, 1984). It is widely used in various cancer therapies such as breast cancer, ovarian cancer, stomach cancer and non-small cell lung cancer. Various clinical studies are currently in progress regarding the optimum dosage and dosing schedule for paclitaxel therapy. One of the major adverse side effects of paclitaxel therapy is granulocytopenia, and the dosage is restricted due to the occurrence of this adverse side effect (see, for example, Seidmann, A.D., et al., Dose-dense therapy with weekly 1-hour paclitaxel infusions in the treatment of metastatic breast cancer, J. Clin. Oncol., 16: 3353-3361, 1998).

Studies are being conducted on a method for lowering adverse side effects of paclitaxel therapy in which the drug to be used and its dosage are determined by predicting adverse side effects by analyzing a patient's genes. This research has clearly demonstrated the possibility of genes related to drug metabolism or genes functionally related to drug activity being the cause of the occurrence of the adverse side effects of a drug. For example, it has been shown that several cSNPs in genes involved in drug metabolism such as the cytochrome P450 family are involved at a high frequency with the adverse side effects of several drugs (Relling, M.V. and Dervieux, T., Pharmacogenetics and cancer therapy, Nat. Rev. Cancer, I : 99-108,2001). However, since the allele frequency of these high-risk genetic polymorphisms is comparatively low, they are inadequate for explaining the possibility of the occurrence of adverse side effects for the majority of patients. Thus, there is a need for further research on the correlation between the occurrence of adverse side effects caused by paclitaxel therapy and genetic polymorphisms.

Japanese Patent Application Laid-open No. 2003-93068 discloses a method for predicting the susceptibility to paclitaxel of patients by investigating polymorphism of the metabolizing enzyme CYP2C8. However, all of the genetic mutations accompanying amino acid mutations disclosed in this application have a low allele frequency. For example, the inventors of this application reported these allele frequencies to be less than 0.007 (Soyama, A., Y. Saito, et al. (2001), "Non-synonymous single nucleotide alterations found in the CYP2C8 gene result in reduced in vitro paclitaxel metabolism", Biol. Pharm. Bull. 24(12), 1427-1430).

An object of the present invention is to provide a method and kit for predicting the possibility of the occurrence of granulocytopenia in paclitaxel therapy.

### DISCLOSURE OF THE INVENTION

The present invention provides a method for predicting the risk of the occurrence of granulocytopenia caused by paclitaxel therapy in a subject comprising identifying in a gene isolated from the subject one or more genetic polymorphisms selected from the group consisting of:
a genetic polymorphism at the 11 th nucleotide of the sequence defined by SEQ ID NO: 6 in BUB1b gene,
a genetic polymorphism at the 11 th nucleotide of the sequence defined by SEQ ID NO: 7 in BUB1b gene,
a genetic polymorphism at the 11 th nucleotide of the sequence defined by SEQ ID NO: 8 in BUB1b gene,
a genetic polymorphism at the 11th nucleotide of the sequence defined by SEQ ID NO: 9 in BUB1b gene, and
a genetic polymorphism at the 11 th nucleotide of the sequence defined by SEQ ID NO: 10 in BUB1b gene. These sequences are shown in Table 1.

**Table 1**

| JSNP ID | Sequence | SEQ ID NO: |
|---|---|---|
| IMS-JST111898 | | 1 |
| IMS-JST105874 | TACTTTTACCYTAAATATGAG | 2 |
| IMS-JST082397 | GAGATCAGTARAAACAGTATG | 3 |
| IMS-JST071852 | GAAATTTCCAWAGTGCTGGTT | 4 |
| IMS-JST071853 | ATTGCTATTTRTCCATGATCA | 5 |
| IMS-JST074538 | | 6 |
| IMS-JST079837 | GACTGACACAKAATTATTATT | 7 |
| IMS-JST044164 | | 8 |
| IMS-JST063023 | AGGAAGGCAAYCTGTTTTTTT | 9 |
| IMS-JST042569 | GGGTACATCTYAGCTATGCCA | 10 |

| | | |
|---|---|---|
| R=A/G; Y=T/C; W=T/A; K=T/G | | |

In one aspect of the present invention, the risk of the occurrence of granulocytopenia is predicted to be high when the genotype at the 11th nucleotide of the sequence defined by SEQ ID NO: 6 in BUB I b gene is A/A, and the risk of the occurrence of granulocytopenia is predicted to be low when the genotype is A/G or G/G

In another aspect of the present invention, the risk of the occurrence of granulocytopenia is predicted to be high when the genotype at the 11th nucleotide of the sequence defined by SEQ ID NO: 7 in BUB1b gene is T/T, and the risk of the occurrence of granulocytopenia is predicted to be low when the genotype is G/T or G/G.

In another aspect of the present invention, the risk of the occurrence of granulocytopenia is predicted to be high when the genotype at the 11 th nucleotide of the sequence defined by SEQ ID NO: 8 in BUB1b gene is C/C, and the risk of the occurrence of granulocytopenia is predicted to be low when the genotype is C/T or T/T.

In another aspect of the present invention, the risk of the occurrence of granulocytopenia is predicted to be high when the genotype at the 11th nucleotide of the sequence defined by SEQ ID NO: 9 in BUB1b gene is C/C, and the risk of the occurrence of granulocytopenia is predicted to be low when the genotype is C/T or T/T.

In another aspect of the present invention, the risk of the occurrence of granulocytopenia is predicted to be high when the genotype at the 11 th nucleotide of the sequence defined by SEQ ID NO: 10 in BUB1b gene is T/T, and the risk of the occurrence of granulocytopenia is predicted to be low when the genotype is C/T or C/C.

Moreover, according to the present invention, the risk of the occurrence of granulocytopenia can be predicted with higher accuracy by combining one or more SNPs identified by the present invention. As will be indicated in the examples described later, the correlation between a specific combination of SNPs and the incidence of granulocytopenia was found to be particularly high. Namely, the present invention provides a method for predicting the risk of the occurrence of granulocytopenia caused by paclitaxel therapy in a subject comprising identifying genetic polymorphisms at the 11th nucleotide of the sequence defined by SEQ ID NO: 4 in CYP2C8 gene and the 11th nucleotide in the sequence defined by SEQ ID NO: 6 in BUB1b gene in the gene isolated from the subject.

The risk of the occurrence of granulocytopenia is preferably predicted to be high when the genotype at the 11th nucleotide in the sequence defined by SEQ ID NO: 4 in CYP2C8 gene is T/T, and the genotype at the 11th nucleotide in the sequence defined by SEQ ID NO: 6 in BUB1b gene is A/A or G/G. In addition, the risk of the occurrence of granulocytopenia is preferably predicted to be low when the genotype at the 11th nucleotide in the sequence defined by SEQ ID NO: 4 in CYP2C8 gene is A/T or A/A, and the genotype at the 11th nucleotide in the sequence defined by SEQ ID NO: 6 in BUB1b gene is A/G.

In still another aspect, the present invention provides a diagnostic kit for predicting the risk of the occurrence of granulocytopenia caused by paclitaxel therapy in a patient. The kit comprises a reagent for identifying in a gene isolated from the patient one or more genetic polymorphisms selected from the group consisting of:
a genetic polymorphism at the 11th nucleotide of the sequence defined by SEQ ID NO: 6 in BUB1b gene,
a genetic polymorphism at the 11th nucleotide of the sequence defined by SEQ ID NO: 7 in BUB I b gene,
a genetic polymorphism at the 11th nucleotide of the sequence defined by SEQ ID NO: 8 in BUB1b gene,
a genetic polymorphism at the 11th nucleotide of the sequence defined by SEQ ID NO: 9 in BUB1b gene, and
a genetic polymorphism at the 11th nucleotide of the sequence defined by SEQ ID NO: 10 in BUB1b gene.

The reagent contained in the kit of the present invention is preferably one or more nucleic acid molecules selected from the following nucleic acid molecules: a nucleic acid molecule having:
a nucleic acid molecule having:
   a sequence of at least 10 contiguous nucleotides containing the 11th nucleotide of the sequence defined by SEQ ID NO: 6 in BUB1b gene, or a sequence complementary thereto; or
   a sequence of at least 10 contiguous nucleotides adjacent to the 11th nucleotide of the sequence defined by SEQ ID NO: 6 in BUB1b gene, or a sequence complementary thereto;
a nucleic acid molecule having:
   a sequence of at least 10 contiguous nucleotides containing the 11th nucleotide of the sequence defined by SEQ ID NO: 7 in BUB1b gene, or a sequence complementary thereto; or
   a sequence of at least 10 contiguous nucleotides adjacent to the 11th nucleotide of the sequence defined by SEQ ID NO: 7 in BUB1b gene, or a sequence complementary thereto;
a nucleic acid molecule having:
   a sequence of at least 10 contiguous nucleotides containing the 11th nucleotide of the sequence defined by SEQ ID NO: 8 in BUB1b gene, or a sequence complementary thereto; or
   a sequence of at least 10 contiguous nucleotides adjacent to the 11h nucleotide of the sequence defined by SEQ ID NO: 8 in BUB1b gene, or a sequence complementary thereto;
a nucleic acid molecule having:
   a sequence of at least 10 contiguous nucleotides containing the 11 th nucleotide of the sequence defined by SEQ ID NO: 9 in BUB1b gene, or a sequence complementary thereto; or
   a sequence of at least 10 contiguous nucleotides adjacent to the 11th nucleotide of the sequence defined by SEQ ID NO: 9 in BUB1b gene, or a sequence complementary thereto; and
a nucleic acid molecule having:
   a sequence of at least 10 contiguous nucleotides containing the 11th nucleotide of the sequence defined by SEQ ID NO: 10 in BUB1b gene, or a sequence complementary thereto; or
   a sequence of at least 10 contiguous nucleotides adjacent to the 11th nucleotide of the sequence defined by SEQ ID NO: 10 in BUB1b gene, or a sequence complementary thereto.

As used herein, the phrase "a nucleic acid molecule contains a nucleotide" means that a nucleotide corresponding to the target SNP site is contained in the sequence of the nucleic acid molecule, and this nucleotide may be located within the nucleic acid molecule or at the 5'- or 3'-end. This type of nucleic acid molecule can be used in SNP typing as a hybridization probe or TaqMan probe. Moreover, a nucleic acid molecule of the present invention may further comprise a sequence that is unrelated to the region around the SNP site. This type of nucleic acid molecule can be used in SNP typing as a primary probe in the invader method. In addition, a nucleic acid molecule being "adjacent" to a nucleotide refers to the nucleic acid molecule not containing a nucleotide corresponding to the target SNP site, but rather containing an upstream or downstream contiguous nucleotide sequence that is adjacent to the SNP site. An example of a sequence "adjacent" to the 11th nucleotide in the sequence defined by SEQ ID NO: 1 is a sequence that contains nucleotide nos. 1 to 10 in the sequence defined by SEQ ID NO: 1, while another example is a sequence that contains nucleotide nos. 12 to 21. This type of nucleic acid molecule can be used in SNP typing as an invader probe in the invader method or as a primer in the MALDI-TOF/MS method and primer extension method. These probes can be designed by referring to the sequence in CYP2C8 gene or BUB1b gene according to the teaching of the present invention.

In addition, the reagent contained in the kit of the present invention preferably comprises one or more nucleic acid molecules selected from the following primer nucleic acid molecules:
a PCR primer pair designed so as to amplify DNA corresponding to the region containing the 11th nucleotide of the sequence defined by SEQ ID NO: 6 in BUB1b gene;
a PCR primer pair designed so as to amplify DNA corresponding to the region containing the 11th nucleotide of the sequence defined by SEQ ID NO: 7 in BUB1b gene;
a PCR primer pair designed so as to amplify DNA corresponding to the region containing the 11th nucleotide of the sequence defined by SEQ ID NO: 8 in BUB1b gene;
a PCR primer pair designed so as to amplify DNA corresponding to the region containing the 11th nucleotide of the sequence defined by SEQ ID NO: 9 in BUB1b gene; and,
a PCR primer pair designed so as to amplify DNA corresponding to the region containing the 11th nucleotide of the sequence defined by SEQ ID NO: 10 in BUB1b gene.

These primer pairs can be used to amplify a target gene in various typing methods. These primer pairs can be designed by referring to the sequence in CYP2C8 gene or BUB1b gene according to the teaching of the present invention. Methods for designing primer pairs suitable for amplification are well known in the art.

### DETAILED DESCRIPTION OF THE INVENTION

In the present invention, 298 genes related to drug metabolism and genes pharmaceutically related to the mechanism of action of paclitaxel were selected to search for genetic polymorphisms associated with adverse side effects of paclitaxel therapy. Next, search was made for SNPs present in these 298 genes in the JSNP database, and 2,727 SNPs were identified. DNA was extracted from the peripheral blood of 54 breast cancer patients administered paclitaxel, and subjected to SNPs typing. A correlation with the occurrence of granulocytopenia was found for five SNPs mapped within CYP2C8 gene (IMS-JST111898 (SEQ ID NO: 1), IMS-JST105874 (SEQ ID NO: 2), IMS-JST082397 (SEQ ID NO: 3), IMS-JST071852 (SEQ ID NO: 4) and IMS-JST071853 (SEQ ID NO: 5)) and five SNPs mapped within BUB1b gene (IMS-JST074538 (SEQ ID NO: 6), IMS-JST079837 (SEQ ID NO: 7), IMS-JST044164 (SEQ ID NO: 8), IMS-JST 063023 (SEQ ID NO: 9) and IMS-JST042569 (SEQ ID NO: 10)) as are indicated in the examples to be described later.

The above-mentioned IMS-JST numbers refer to the entry numbers in the JSNP database (http://snp.ims.u-tokyo.ac.jp/), and can be accessed from various databases including the dbSNP database of NCBI. In the specification, the SNP sites in the genome sequences along with those sequences containing 10 nucleotides upstream and downstream of those SNP sites are indicated as SEQ ID NOs: 1 to 10 to clarify the locations of these SNPs found in the present invention, and the SNPs are represented herein with reference to these sequence ID numbers. It will be clear to a person with ordinary skill in the art that a portion of the nucleotide sequences indicated with these sequence ID numbers may subsequently be changed as a result of a sequencing error or the discovery of new polymorphisms.

CYP2C8 (cytochrome P450, family 2, subfamily C, polypeptide 8) is a cytochrome P450 involved in the metabolism of various drugs, and is mapped to chromosome location 10q23.33. Metabolism of paclitaxel mainly takes place in hepatocytes, and its metabolites are excreted into the bile. The hydrolytic ability of CYP2C8 has an important function in the detoxification of paclitaxel, and paclitaxel is decomposed to the detoxified form, 6α-hydroxypaclitaxel as a result of this action (Rahman, A., et al.: Selective biotransformation of taxol to 6α-hydroxytaxol by human cytochrome P450 2C8, Cancer Res., 54: 5543-5546, 1994). Consequently, CYP2C8 protein is believed to have an important function in determining the concentration of paclitaxel in the blood, suggesting that CYP2C8 is also related to adverse side effects. CYP2C8 gene is known to have several cSNPs. For example, one of the cSNPs occurring at 5 locations accompanying amino acid substitution (416G -> A) is known to have a slower rate of paclitaxel metabolism than the wild type (Bahadur, N., et al., CYP2C8 polymorphisms in Caucasians and their relationship with paclitaxel 6alpha-hydroxylase activity in human liver microsomes, Biochem. Pharmacol., 64: 1579-1589, 2002; Dai, D., et al., Polymorphisms in human CYP2C8 decrease metabolism of the anticancer drug paclitaxel and arachidonic acid, Pharmacogenetics, 11: 597-607, 2001). However, according to analytical research on allele frequency in Japanese population, the frequency of these cSNPs is known to be extremely low. In actuality, only one 1196A -> G mutant allele was found among 54 specimens in typing conducted in the present invention. On the basis of these findings, known cSNPs present in CYP2C8 gene are suggested to hardly be involved at all in granulocytopenia associated with paclitaxel. The SNPs found by the present invention are thought to be related to granulocytopenia by a yet unknown mechanism.

BUB1b gene is a homologue of BUB gene that is related to a checkpoint of mitosis in budding yeast, and is mapped to a chromosome location of 15q15 (Cahill, D.P. et al., Mutations of mitotic checkpoint genes in human cancers, Nature, 392: 300-303, 1998; Hoyt, M.A. et al., S. cerevisiae genes required for cell cycle arrest in response to loss of microtubule function, Cell, 66: 507-517, 1991; Li, R. and Murray, A.W., Feedback control of mitosis in budding yeast, Cell, 66: 519-531, 1991). The antitumor effects of paclitaxel are exerted by promoting microtubule polymerization, and microtubules are the target molecules of paclitaxel. These findings suggest that there is a functional relationship between BUB1b gene and the pharmacological action of paclitaxel.

Information on the locations and polymorphisms of SNPs that have been found to be related to adverse side effects associated with paclitaxel therapy are shown in the following tables.

**Table 2**

| |
|---|
| IMS-JST 111898 |
| General Information |
| JSNP IQ: IMS-JSTII1898 |
| dbSNP ID (rs#): 1557044 |
| dbSNP ID (ss#): 4944611 |
| HGVbase ID: SNP000830254 |
| Organism: Homo sapiens |
| Molecular type: Genomic |
| Allele Sequence |
| Variation Type: SNP |
| Flanking Sequence Information (SEQ ID NO: 11) |
| |
| Observed: A/G |
| |

**Table 3**

| |
|---|
| 1MS-JST105874 |
| General Information |
| JSNP ID: IMS-JST105874 |
| dbSNP ID (rs#): 3752988 |
| dbSNP ID (ss#): 4939017 |
| HGVbase ID: |
| Organism: Homo sapiens |
| Molecular type: Genomic |
| Allele Sequence |
| Variation Type: SNP |
| Flanking Sequence Information (SEQ ID NO: 12) |
| |
| Observed: T/C |
| |

**Table 4**

| |
|---|
| IMS-JST082397 |
| General Information |
| JSNP ID: IMS-JST082397 |
| dbSNP ID (rs#): 1891071 |
| cbSNP ID (ss#): 4923304 |
| HGVbase ID: |
| Organism: Homo sapiens |
| Molecular type: Genomic |
| Allele Sequence |
| Variation Type: SNP |
| Flanking Sequence Information (SEQ ID NO: 13) |
| |
| Observed: A/G |
| |

**Table 5**

| |
|---|
| IMS-JST071852 |
| General Information |
| JSNP ID: IMS-JST071852 |
| dbSNP ID (rs#): 2275620 |
| cbSNP ID (ss#): 3211768 |
| HGVbase ID: SNP001282389 |
| Organism: Homo sapiens |
| Molecular type: Genomic |
| Allele Sequence |
| Variation Type: SNP |
| Flanking Sequence Information (SEQ ID NO: 14) |
| |
| Observed: T/A |
| |

**Table 6**

| |
|---|
| IMS-JST071853 |
| General Information |
| JSNP ID: IMS-JST071853 |
| dbSNP ID (rs#): 1934951 |
| cbSNP ID (ss#): 3211769 |
| HGVbase ID: SNP001276002 |
| Organism: Homo sapiens |
| Molecular type: Genomic |
| Allele Sequence |
| Variation Type: SNP |
| Flanking Sequence Information (SEQ ID NO: 15) |
| |
| Observed: G/A |
| |

**Table 7**

| |
|---|
| IMS-JST074538 |
| General Information |
| JSNP ID: IMS-JST074538 |
| dbSNP ID (rs#): 2277559 |
| cbSNP ID (ss#): 3214454 |
| HGVbase ID: SNP001383307 |
| Organism: Homo sapiens |
| Molecular type: Genomic |
| Allele Sequence |
| Variation Type: SNP |
| Flanking Sequence Information (SEQ ID NO: 16) |
| |
| Observed: A/G |
| |

**Table 8**

| |
|---|
| IMS-JST079837 |
| General Information |
| JSNP ID: IMS-JST079837 |
| dbSNP ID (rs#): 3214012 |
| cbSNP ID (ss#): 4474916 |
| HGVbase ID: |
| Organism: Homo sapiens |
| Molecular type: Genomic |
| Allele Sequence |
| Variation Type: SNP |
| Flanking Sequence Information (SEQ ID NO: 17) |
| |
| Observed: T/G |
| |

**Table 9**

| |
|---|
| IMS-JST044164 |
| General Information |
| JSNP ID: IMS-JST044164 |
| dbSNP ID (rs#): 1801376 |
| cbSNP ID (ss#): 3234079 |
| HGVbase ID: |
| Organism: Homo sapiens |
| Molecular type: Genomic |
| Allele Sequence |
| Variation Type: SNP |
| Flanking Sequence Information (SEQ ID NO: 18) |
| |
| Observed: C/T |
| |

**Table 10**

| |
|---|
| IMS-JST063023 |
| General Information |
| JSNP ID: IMS-JST063023 |
| dbSNP ID (rs#): 2305653 |
| cbSNP ID (ss#): 3252938 |
| HGVbase ID: SNP001393945 |
| Organism: Homo sapiens |
| Molecular type: Genomic |
| Allele Sequence |
| Variation Type: SNP |
| Flanking Sequence Information (SEQ ID NO: 19) |
| |
| Observed: C/T |
| |

**Table 11**

| |
|---|
| IMS-JST042569 |
| General Information |
| JSNP ID: IMS-JST042569 |
| dbSNP ID (rs#): 2290551 |
| cbSNP ID (ss#): 3232484 |
| HGVbase ID: SNP001383051 |
| Organism: Homo sapiens |
| Molecular type: Genomic |
| Allele Sequence |
| Variation Type: SNP |
| Flanking Sequence Information (SEQ ID NO: 20) |
| |
| Observed: T/C |
| |

In the method of the present invention, peripheral blood, other body fluid, cells or tissue and so forth is collected from subjects scheduled to be treated with paclitaxel or are currently being administered paclitaxel, then genomic DNA is prepared from these samples in accordance with established methods. When necessary, a sequence at a site to be typed is amplified. Typing of genetic polymorphisms can be easily carried out using various methods known or being developed in the art. Examples of typing methods include, but are not limited to, the direct sequencing method, invader method, TaqMan method, MALDI-TOF/MS method, primer extension method and hybridization method.

In the case of using the direct sequencing method, DNA of the region that contains the SNP site is amplified by PCR, and the SNP can be identified by directly sequencing the sequence of the PCR product.

In the case of using the invader method, an invader probe containing a sequence specific to the region 3'to the SNP site, and a primary probe containing a sequence specific to the region 5'to the SNP site of a template and an unrelated flap sequence, are prepared. Cleavase is then allowed to act in the presence of these probes, a FRET probe containing a sequence complementary to the flap sequence and an auto-complementary sequence that is labeled with both a fluorescent dye and a quencher, and the template. When the primary probe hybridizes with the template, the 3' end of the invader probe penetrates the SNP site, and this structure is cleaved by the Cleavase resulting in dissociation of the flap. The flap binds to the FRET probe and the fluorescent dye portion is cleaved by the Cleavase resulting in emission of fluorescence. By preparing two sets of the flap-FRET probe labeled with different fluorescent dyes, each homozygote and heterozygote can be distinguished in a single assay.

In the case of using the TaqMan method, an allele-specific probe labeled with a fluorescent dye and a quencher is hybridized to a target site, and PCR is carried out using a primer designed to amplify the region containing this site. Simultaneous to progression of the elongation reaction from the primer, the hybridized probe is cleaved by the 5' exonuclease activity ofTaqDNA polymerase. Fluorescence is generated when the fluorescent dye is separated from the quencher and SNP can be identified by detecting this fluorescence.

In the case of using the MALDI-TOF/MS method, a primer adjacent to an SNP site is prepared, and primer extension is carried out using the sample DNA amplified by PCR as a template to elongate the primer by one nucleotide with ddNTP. The added ddNTP is identified by mass spectrophotometry with MALDI-TOF/MS.

In the case of using the hybridization method, DNA of the region containing the SNP site is amplified by PCR and the amplification product is detected by hybridization using a probe specific to the SNP site. Moreover, various other methods such as the RFLP method, DNA chip method, molecular beacon method and ligation method have been developed in addition to the above-mentioned methods, and any of these can be used in the present invention.

According to the method of the present invention, the risk of the occurrence of adverse side effects caused by paclitaxel therapy can be predicted by typing one or more SNP sites identified in the present invention and referring to statistical data indicated in the examples to be described later. The method of the present invention is preferably applied to mongoloids and particularly preferably applied to Japanese. More preferably, the risk of the occurrence of adverse side effects caused by paclitaxel therapy is predicted by typing SNP in CYP2C8 gene and typing SNP in BUB1b gene identified in the present invention and combining those results. Examples of such combinations are listed below:
IMS-JST111898 (SEQ ID NO: 1) and IMS-JST074538 (SEQ ID NO: 6),
IMS-JST111898 (SEQ ID NO: 1) and IMS-JST079837 (SEQ ID NO: 7),
IMS-JST111898 (SEQ ID NO: 1) and IMS-JST044164 (SEQ ID NO: 8),
IMS-JST111898 (SEQ ID NO: 1) and IMS-JST063023 (SEQ ID NO: 9),
IMS-JST111898 (SEQ ID NO: 1) and IMS-JST042569 (SEQ ID NO: 10),
IMS-JST105874 (SEQ ID NO: 2) and IMS-JST074538 (SEQ ID NO: 6),
IMS-JST105874 (SEQ ID NO: 2) and IMS-JST079837 (SEQ ID NO: 7),
IMS-JST105874 (SEQ ID NO: 2) and IMS-JST044164 (SEQ ID NO: 8),
IMS-JST105874 (SEQ ID NO: 2) and IMS-JST063023 (SEQ ID NO: 9),
IMS-JST105874 (SEQ ID NO: 2) and IMS-JST042569 (SEQ ID NO: 10),
IMS-JST082397 (SEQ ID NO: 3) and IMS-JST074538 (SEQ ID NO: 6),
IMS-JST082397 (SEQ ID NO: 3) and IMS-JST079837 (SEQ ID NO: 7),
IMS-JST082397 (SEQ ID NO: 3) and IMS-JST044164 (SEQ ID NO: 8),
IMS-JST082397 (SEQ ID NO: 3) and IMS-JST063023 (SEQ ID NO: 9),
IMS-JST082397 (SEQ ID NO: 3) and IMS-JST042569 (SEQ ID NO: 10),
IMS-JST071852 (SEQ ID NO: 4) and IMS-JST074538 (SEQ ID NO: 6),
IMS-JST071852 (SEQ ID NO: 4) and IMS-JST079837 (SEQ ID NO: 7),
IMS-JST071852 (SEQ ID NO: 4) and IMS-JST044164 (SEQ ID NO: 8),
IMS-JST071852 (SEQ ID NO: 4) and IMS-JST063023 (SEQ ID NO: 9),
IMS-JST071852 (SEQ ID NO: 4) and IMS-JST042569 (SEQ ID NO: 10),
IMS-JST071853 (SEQ ID NO: 5) and IMS-JST074538 (SEQ ID NO: 6),
IMS-JST071853 (SEQ ID NO: 5) and IMS-JST079837 (SEQ ID NO: 7),
IMS-JST071853 (SEQ ID NO: 5) and IMS-JST044164 (SEQ ID NO: 8),
IMS-JST071853 (SEQ ID NO: 5) and IMS-JST063023 (SEQ ID NO: 9),
IMS-JST071853 (SEQ ID NO: 5) and IMS-JST042569 (SEQ ID NO: 10).

The present invention also provides a kit for predicting the risk of the occurrence of adverse side effects caused by paclitaxel therapy comprising a reagent for use in the above-mentioned typing method. Examples of the reagent include a probe and a primer. Primers used to amplify a region of a gene that contains the SNP site identified in the present invention are preferably 15 to 30 nucleotides in length, and are designed to flank the target SNP site so that an amplification product of a desired length is formed by the PCR. A primary probe used in the invader method contains a sequence specific to a target region of the 5'side from the target SNP site and also contains an unrelated flap sequence. In addition, an invader probe used in the invader method as well as a primer used in the MALDI-TOF/MS method and primer extension method do not contain the nucleotide just corresponding to the target SNP site, but contain an upstream or downstream contiguous nucleotide sequence adjacent to the SNP site. The design methods and synthesis methods of such probes and primers are well known in the art.

The contents of all of the patents and reference documents explicitly cited in the present specification are incorporated herein by reference. All of the contents described in the specification and drawings of Japanese Patent Application No. 2003-375369, which serves as the basis for claiming priority by the present application, are incorporated herein by reference.

### EXAMPLES

The following Examples provide a more detailed explanation of the present invention, although these examples do not limit the scope of the present invention.

### Clinical Samples

54 breast cancer patients were registered in a clinical study of paclitaxel preoperative chemotherapy. The following summarizes the criteria for patient selection. 1) Breast cancer patients age 70 or younger in stage II or IIIa as indicated by histopathological testing; and, 2) suitable physiological function (WBC > 4,000 mm³, platelet count > 10,000 mm³, hemoglobin level > 10 g/dl, serum creatinine concentration < 1.2 mg/dl, serum total bilirubin level < 1.5 mg/dl, GOT/GPT < 60/70). Patients who received proceeding chemotherapy or radiation therapy were excluded. Namely, all 54 patients who participated in this clinical study of paclitaxel preoperative chemotherapy were nearly at the same stage either clinically or histologically, and had no prior history of chemotherapy. The patients received a dose of 80 mg/m² of paclitaxel by intravenous infusion over the course of 1 hour. Administration was repeated once a week for 12 weeks. The most frequently observed adverse side effect among these patients was granulocytopenia, and this was observed in 24 patients.

### Definition of Adverse Side Effects

White blood cell count, red blood cell count, hemoglobin level and platelet count were measured weekly for each patient to evaluate the presence or absence of adverse side effects. Adverse side effects observed during the course of therapy were evaluated and graded based on the Common Toxicity Criteria of the US National Cancer Institute (NCI-CTC) (see Trotti, A., et al., Common toxicity criteria: version 2.0 - an improved reference for grading the acute effects of cancer treatment: impact on radiotherapy, Int. J. Radiat. Oncol. Biol. Phys., 47: 13-47, 2000). All clinical data was collected anonymously using the SCTS21 System (Mitsui Knowledge Industry) and used in subsequent analyses. In the grading of granulocytopenia, patients demonstrating a grade of 1 to 4 of the NCI-CTC classification were evaluated as having granulocytopenia, while patients demonstrating a grade of 0 were evaluated as not having granulocytopenia. There were no correlations observed with respect to age or age of onset between the granulocytopenia group and non-granulocytopenia group.

### Typing of SNPs

In order to search for genetic polymorphisms associated with adverse side effects of paclitaxel, 298 genes were first selected that related to drug metabolism or are pharmacologically related to the mechanism of action of paclitaxel. Next, a search was made of SNPs present in these 298 genes using the JSNP database, and 2,727 SNPs were selected.

SNPs present at 2,727 locations in the 298 genes were typed using the invader method for the 54 patients in this study. 14 ml of peripheral blood were collected from the 54 patients. A standard method was used to extract DNA from the peripheral blood. Before typing with the invader method, roughly 500 bp portions around the target SNP sites were amplified by PCR, where 48 DNA fragments were simultaneously amplified by carrying out multiplex PCR using 10 ng of DNA as template and 48 sets of primers. The primers used to amplify the region around each SNP site were designed based on the sequences described in the JSNP database. PCR was carried out using the following composition: 6.7 mM MgCl₂, 67 mM TrisHCl, 16.6 mM NH₄SO₄, 10 mM 2-mercaptoethanol, 6.7 µM EDTA, 15 mM dNTPs, 10% DMSO, 1 pmol of each primer and 0.05 U Ex-Taq. PCR comprised of the initial denaturing reaction for 2 minutes at 94°C followed by 35 cycles of 15 seconds at 94°C, 15 seconds at 60°C and 2 minutes at 72°C. After diluting the PCR product with sterile distilled water using the Multimek96 reaction robot, the diluted products were dispensed into an invader reaction card using the TANGO dispenser. Next, the invader reaction reagent was dispensed into the invader reaction card using a Cartesian dispenser. The invader reaction reagent contained an allele-specific oligonucleotide, Cleavase VII, and a FRET cassette labeled with FAM or Redmond Red. These reagents were purchased from Third Wave. Fluorescence signals were detected with TECAN Ultra, and genotypes were determined by portraying FAM and Redmond Red signal intensity on a two-dimensional chart.

The genotype of 2,123 SNPs out of the 2,727 SNPs could be determined in 80% or more of the samples tested. In order to assess genotype accuracy, the typing data of three randomly selected SNPs were compared with genotypes determined by the RLFP method. The results were the same in both typing methods for all of more than about 1,000 genotypes tested, suggesting that the typing accuracy was extremely high. In addition, each SNP was examined for Hardy-Weinberg equilibrium by chi-square test, and all of the SNPs tested were suggested to be at Hardy-Weinberg equilibrium.

### Search and Correlation Analysis for SNPs Associated with Adverse Side Effects

First, the haplotype block structure was constructed for each gene. The linkage disequilibrium coefficient |D'| between two arbitrary SNPs was estimated for every combination of SNPs mapped within the same gene, and a matrix was prepared in which the results were arranged in order of the locations of the SNPs. If the value of |D'| between two SNPs was 0.9 or more, a haplotype block was presumed to have been formed between two genes. As a result, the 298 genes that were typed were found to be subdivided into 419 haplotype blocks.

Next, two-stage screening was carried out in order to identify those SNPs associated with adverse side effects. In the first stage, independency was tested using a 2 x 3 contingency table for the distribution of genotypes between an adverse side effect group and a non-adverse side effect group. As a result of the first-stage of screening, two haplotype blocks were found to be correlated with granulocytopenia. These haplotype blocks respectively contained 5 SNPs mapped in CYP2C8 gene and 5 SNPs mapped in BUB1b gene. The minimum p value in the haplotype block containing CYP2C8 gene was 0.0065, while the minimum p value in the haplotype block containing BUB1b gene was 0.010.

Those SNPs identified in the first stage of screening which were present in the same haplotype block or within the same gene and which had a p value of 0.05 or less were used in the second stage analysis. In the second stage, a 2 x 2 contingency table was prepared on the basis of a dominant gene model or recessive gene model, and independency was tested using Fisher's exact test.

**Table 12 Correlation Between CYP2C8 Gene and Granulocytopenia**

| Distance(bp) | SNP | Genotype | Granulocytopenia | | P value | Odds ratio |
|---|---|---|---|---|---|---|
| | | | (+)(n=24) | (-)(n=30) | | (95%c.i.) |
| | | | | | | |
| 0 | IMS-JST111898 | G/G | 9 | 2 | 0.00774 | 8.13 |
| | | A/G & A/A | 15 | 27 | | (1.46-45.5) |
| | | | | | | |
| 6,506 | IMS-JST105874 | T/T | 13 | 3 | 0.00351 | 7.63 |
| | | C/T & C/C | 11 | 27 | | (1.72-33.3) |
| | | | | | | |
| 26,018 | IMS-JST082397 | G/G | 10 | 2 | 0.00271 | 10.0 |
| | | A/G & A/A | 14 | 28 | | (1.93-52.6) |
| | | | | | | |
| 28,791 | IMS-JST071852 | T/T | 10 | 2 | 0.00202 | 10.7 |
| | | A/T & A/A | 13 | 28 | | (2.09-55.6) |
| | | | | | | |
| 32,841 | IMS-JST071853 | G/G | 11 | 3 | 0.00351 | 7.63 |
| | | A/G & A/A | 13 | 27 | | (1.72-33.3) |

**Table 13 Correlation Between BUB1b Gene and Granulocytopenia**

| Dis tance(bp ) | SNP | Genotype | Granulocytopenia | | P value | Odds ratio |
|---|---|---|---|---|---|---|
| | | | (+)(n=24) | (-)(n=30) | | (95%c.i.) |
| | | | | | | |
| 0 | IMS-JST074538 | A/A | 14 | 7 | 0.00627 | 5.11 |
| | | A/G & G/G | 9 | 23 | | (1.41-18.5) |
| | | | | | | |
| 3,822 | IMS-JST079837 | T/T | 14 | 7 | 0.00627 | 5.11 |
| | | G/T & G/G | 9 | 23 | | (1.41-18.5) |
| | | | | | | |
| 24,524 | IMS-JST044164 | C/C | 14 | 10 | 0.0187 | 3.85 |
| | | C/T & T/T | 8 | 22 | | (1.93-52.6) |
| | | | | | | |
| 41,191 | IMS-JST063023 | C/C | 15 | 9 | 0.0111 | 4.36 |
| | | C/T & T/T | 8 | 21 | | (1.22-15.6) |
| | | | | | | |
| 56,293 | IMS-JST042569 | T/T | 15 | 9 | 0.0169 | 3.89 |
| | | C/T & C/C | 9 | 23 | | (1.10-13.6) |

In the second stage analysis, a higher correlation was observed for all of the SNPs mapped to the two genes than in the case of assuming a recessive gene model. The SNP for which the highest correlation was observed among SNPs mapped to the CYP2C8 gene was IMS-JST071852 (p = 0.0020, odds ratio: 10.7), while that among SNPs mapped to the BUB1b gene was IMS-JST074538 (p = 0.0062, odds ratio: 5.11). In order to investigate whether known cSNPs present at three locations in CYP2C8 gene are related to the SNPs used in this study, the genotypes of the cSNPs at those three locations were determined using the RFLP method. As a result, a heterozygote was only observed at the 1196A -> G site in one patient, while all three of the cSNPs were of the wild type in the other patients. On the basis of these findings, the frequency of the known three cSNPs present in CYP2C8 gene was presumed to be extremely low. In addition, the polymorphism at a single SNP (IMS-JST044164) mapped within BUB1b gene was a cSNP accompanying amino acid substitution (Arg -> Gln). In this study, homozygote of allele having the wild type Arg was shown to be predominant in patients with granulocytopenia.

### Estimation of Probability of Appearance of Adverse Side Effects According to Genotype Combination

The probabilities of the appearance of adverse side effects were calculated for each combination of genotypes for one SNP on CYPC2C8 gene and one SNP on BUB1b gene. A logistic regression model was used for the calculations, where two types of SNP alleletypes on CYP2C8 gene and two types of SNP alleletypes on BUB1b gene were respectively assigned to four variables. A search was made for the most suitable SNP combination using a likelihood ratio test, and the combination of IMS-JST071852 on CYP2C8 gene and IMS-JST074538 on BUB1b gene was selected (p<0.000532). The probabilities of the appearance of adverse side effects for each genotype of these two SNPs are shown in Table 14. The allele frequencies were estimated from the allele frequency of each SNP obtained from the JSNP database.

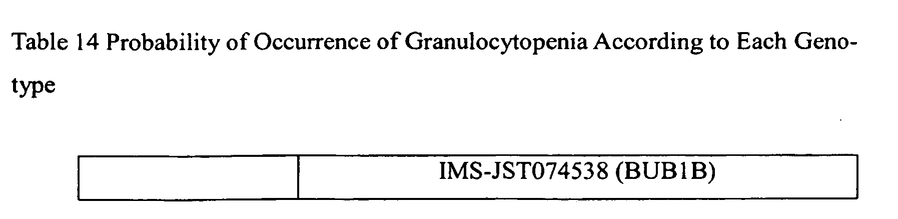

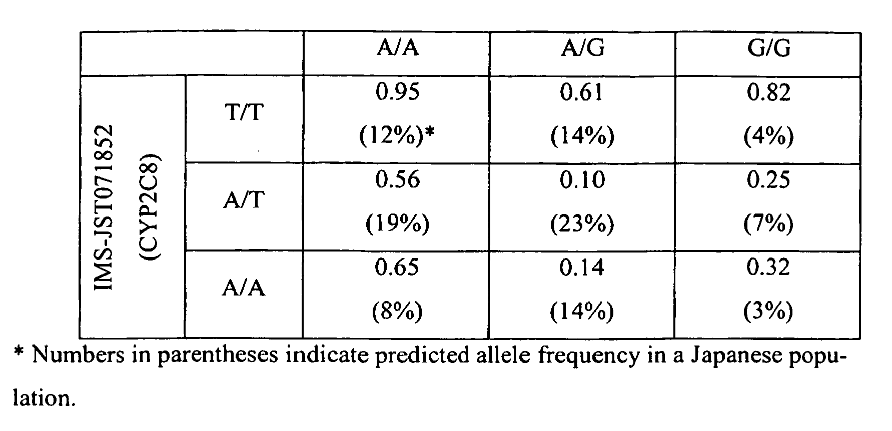

The present invention has shown that the potential for the occurrence of granulocytopenia can be reliably predicted by using two SNPs on two genes. If the combination of the genotypes of IMS-JST071852 on CYP2C8 gene and IMS-JST074538 on BUB1b gene is T/T and A/A or T/T and G/G, then the probability of the occurrence of granulocytopenia is considered to be extremely high. On the basis of the allele frequencies reported in the JSNP database, the frequency of the combinations of these two genotypes among Japanese is 0.12 and 0.04, respectively. On the other hand, the combinations of A/T and A/G or A/A and A/G are considered to represent lower probability of the occurrence of granulocytopenia, and the frequencies of these combinations among Japanese population are 0.23 and 0.14, respectively. Taken together, these results demonstrates that the occurrence of granulocytopenia in paclitaxel therapy can be predicted for roughly half of Japanese population.

## Claims

1. A method for predicting the risk of the occurrence of granulocytopenia caused by paclitaxel therapy in a subject comprising identifying in a gene isolated from the subject one or more genetic polymorphisms selected from the group consisting of:
a genetic polymorphism at the 11 th nucleotide of the sequence defined by SEQ ID NO: 6 in BUB1b gene,
a genetic polymorphism at the 11 th nucleotide of the sequence defined by SEQ ID NO: 7 in BUB1b gene,
a genetic polymorphism at the 11th nucleotide of the sequence defined by SEQ ID NO: 8 in BUB1b gene,
a genetic polymorphism at the 11th nucleotide of the sequence defined by SEQ ID NO: 9 in BUB1b gene, and
a genetic polymorphism at the 11 th nucleotide of the sequence defined by SEQ ID NO: 10 in BUB1b gene.

2. The method according to claim 1, wherein the risk of the occurrence of granulocytopenia is predicted to be high in the case where the gene isolated from the subject is one or more of the following (A) through (E):
(A) the genotype at the 11th nucleotide of the sequence defined by SEQ ID NO: 6 in BUB1b gene is A/A;
(B) the genotype at the 11th nucleotide of the sequence defined by SEQ ID NO: 7 in BUB1b gene is T/T;
(C) the genotype at the 11th nucleotide of the sequence defined by SEQ ID NO: 8 in BUB1b gene is C/C;
(D) the genotype at the 11th nucleotide of the sequence defined by SEQ ID NO: 9 in BUB1b gene is C/C; and
(E) the genotype at the 11th nucleotide of the sequence defined by SEQ ID NO: 10 in BUB1b gene is T/T.

3. The method according to claim 1, wherein the risk of the occurrence of granulocytopenia is predicted to be low in the case where the gene isolated from the subject is one or more of the following (F) through (J):
(F) the genotype at the 11 th nucleotide of the sequence defined by SEQ ID NO: 6 in BUB1b gene is A/G or G/G;
(G) the genotype at the 11 th nucleotide of the sequence defined by SEQ ID NO: 7 in BUB1b gene is G/T or G/G;
(H) the genotype at the 11th nucleotide of the sequence defined by SEQ ID NO: 8 in BUB1b gene is C/T or T/T;
(I) the genotype at the 11th nucleotide of the sequence defined by SEQ ID NO: 9 in BUB1b gene is C/T or T/T; and
(J) the genotype at the 11th nucleotide of the sequence defined by SEQ ID NO: 10 in BUB1b gene is C/T or C/C.

4. The method according to claim 1 comprising identifying in a gene isolated from the subject a genetic polymorphism at the 11th nucleotide of the sequence defined by SEQ ID NO: 4 in CYP2C8 gene, and a genetic polymorphism at the 11th nucleotide of the sequence defined by SEQ ID NO: 6 in BUB1b gene.

5. The method according to claim 4, wherein the risk of the occurrence of granulocytopenia is predicted to be high when the genotype at the 11th nucleotide of the sequence defined by SEQ ID NO: 4 in CYP2C8 gene is T/T, and the genotype at the 11 th nucleotide of the sequence defined by SEQ ID NO: 6 in BUB1b gene is A/A or G/G.

6. The method according to claim 4, wherein the risk of the occurrence of granulocytopenia is predicted to be low when the genotype at the 11 th nucleotide of the sequence defined by SEQ ID NO: 4 in CYP2C8 gene is A/T or A/A, and the genotype at the 11th nucleotide of the sequence defined by SEQ ID NO: 6 in BUB1b gene is A/G.

7. A diagnostic kit for predicting the risk of the occurrence of granulocytopenia caused by paclitaxel therapy in a subject comprising a reagent for identifying in a gene isolated from the subject one or more genetic polymorphisms selected from the group consisting of:
a genetic polymorphism at the 11 th nucleotide of the sequence defined by SEQ ID NO: 6 in BUB1b gene,
a genetic polymorphism at the 11 th nucleotide of the sequence defined by SEQ ID NO: 7 in BUB1b gene,
a genetic polymorphism at the 11 th nucleotide of the sequence defined by SEQ ID NO: 8 in BUB1b gene,
a genetic polymorphism at the 11th nucleotide of the sequence defined by SEQ ID NO: 9 in BUB1b gene, and
a genetic polymorphism at the 11 th nucleotide of the sequence defined by SEQ ID NO: 10 in BUB1b gene.

8. The diagnostic kit according to claim 7, wherein the reagent is one or more nucleic acid molecules selected from the group consisting of:
a nucleic acid molecule having:
a sequence of at least 10 contiguous nucleotides containing the 11th nucleotide of the sequence defined by SEQ ID NO: 6 in BUB1b gene, or a sequence complementary thereto; or
a sequence of at least 10 contiguous nucleotides adjacent to the 11th nucleotide of the sequence defined by SEQ ID NO: 6 in BUB1b gene, or a sequence complementary thereto;
a nucleic acid molecule having:
a sequence of at least 10 contiguous nucleotides containing the 11 th nucleotide of the sequence defined by SEQ ID NO: 7 in BUB1b gene, or a sequence complementary thereto; or
a sequence of at least 10 contiguous nucleotides adjacent to the 11th nucleotide of the sequence defined by SEQ ID NO: 7 in BUB1b gene, or a sequence complementary thereto;
a nucleic acid molecule having:
a sequence of at least 10 contiguous nucleotides containing the 11th nucleotide of the sequence defined by SEQ ID NO: 8 in BUB1b gene, or a sequence complementary thereto; or
a sequence of at least 10 contiguous nucleotides adjacent to the 11th nucleotide of the sequence defined by SEQ ID NO: 8 in BUB1b gene, or a sequence complementary thereto;
a nucleic acid molecule having:
a sequence of at least 10 contiguous nucleotides containing the 11th nucleotide of the sequence defined by SEQ ID NO: 9 in BUB1b gene, or a sequence complementary thereto; or
a sequence of at least 10 contiguous nucleotides adjacent to the 11th nucleotide of the sequence defined by SEQ ID NO: 9 in BUB1b gene, or a sequence complementary thereto; and
a nucleic acid molecule having:
a sequence of at least 10 contiguous nucleotides containing the 11th nucleotide of the sequence defined by SEQ ID NO: 10 in BUB1b gene, or a sequence complementary thereto; or
a sequence of at least 10 contiguous nucleotides adjacent to the 11th nucleotide of the sequence defined by SEQ ID NO: 10 in BUB1b gene, or a sequence complementary thereto.

9. The diagnostic kit according to claim 7, wherein the reagent comprises:
a nucleic acid molecule having:
a sequence of at least 10 contiguous nucleotides containing the 11th nucleotide of the sequence defined by SEQ ID NO: 4 in CYP2C8 gene, or a sequence complementary thereto; or
a sequence of at least 10 contiguous nucleotides adjacent to the 11th nucleotide of the sequence defined by SEQ ID NO: 4 in CYP2C8 gene, or a sequence complementary thereto; and
a nucleic acid molecule having:
a sequence of at least 10 contiguous nucleotides containing the 11 th nucleotide of the sequence defined by SEQ ID NO: 6 in BUB1b gene, or a sequence complementary thereto; or
a sequence of at least 10 contiguous nucleotides adjacent to the 11th nucleotide of the sequence defined by SEQ ID NO: 6 in BUB1b gene, or a sequence complementary thereto.

10. The diagnostic kit according to claim 7, wherein the reagent is one or more PCR primer pairs selected from the group consisting of:
a PCR primer pair designed so as to amplify DNA corresponding to the region containing the 11 th nucleotide of the sequence defined by SEQ ID NO: 6 in BUB1b gene;
a PCR primer pair designed so as to amplify DNA corresponding to the region containing the 11th nucleotide of the sequence defined by SEQ ID NO: 7 in BUB1b gene;
a PCR primer pair designed so as to amplify DNA corresponding to the region containing the 11th nucleotide of the sequence defined by SEQ ID NO: 8 in BUB1b gene;
a PCR primer pair designed so as to amplify DNA corresponding to the region containing the 11th nucleotide of the sequence defined by SEQ ID NO: 9 in BUB1b gene; and,
a PCR primer pair designed so as to amplify DNA corresponding to the region containing the 11th nucleotide of the sequence defined by SEQ ID NO: 10 in BUB1b gene.

11. The diagnostic kit according to claim 7, wherein the reagent comprises:
a PCR primer pair designed so as to amplify DNA corresponding to the region containing the 11th nucleotide of the sequence defined by SEQ ID NO: 4 in CYP2C8 gene; and
a PCR primer pair designed so as to amplify DNA corresponding to the region containing the 11th nucleotide of the sequence defined by SEQ ID NO: 6 in BUB1b gene.

## Patentansprüche

1. Verfahren zum Vorhersagen des Risikos des Auftretens von durch eine Paclitaxeltherapie verursachter Granulozytopänie bei einem Patienten, umfassend das Identifizieren eines genetischen Polymorphismus bzw. mehrerer genetischer Polymorphismen, ausgewählt aus der Gruppe, bestehend aus
einem genetischen Polymorphismus am 11. Nukleotid der der durch SEQ ID NR: 6 definierten Sequenz im BUB1b-Gen,
einem genetischen Polymorphismus am 11. Nukleotid der durch SEQ ID NR: 7 definierten Sequenz im BUB1b-Gen,
einem genetischen Polymorphismus am 11. Nukleotid der durch SEQ ID NR: 8 definierten Sequenz im BUB1b-Gen,
einem genetischen Polymorphismus am 11. Nukleotid der durch SEQ ID NR: 9 definierten Sequenz im BUB1b-Gen,
einem genetischen Polymorphismus am 11. Nukleotid der durch SEQ ID NR: 10 definierten Sequenz im BUB1b-Gen,
in einem von dem Patienten isolierten Gen.

2. Verfahren nach Anspruch 1, wobei das Risiko des Auftretens von Granulozytopänie in dem Falle, in welchem es sich bei dem vom Patienten isolierten Gen um einen oder mehrere der folgenden Punkte (A) bis (E) handelt, hoch ist:
(A) bei dem Genotyp am 11. Nukleotid der durch SEQ ID NR: 6 definierten Sequenz im BUB1b-Gen handelt es sich um A/A;
(B) bei dem Genotyp am 11. Nukleotid der durch SEQ ID NR: 7 definierten Sequenz im BUB1b-Gen handelt es sich um T/T;
(C) bei dem Genotyp am 11. Nukleotid der durch SEQ ID NR: 8 definierten Sequenz im BUB1b-Gen handelt es sich um C/C;
(D) bei dem Genotyp am 11. Nukleotid der durch SEQ ID NR: 9 definierten Sequenz im BUB1b-Gen handelt es sich um C/C; und
(E) bei dem Genotyp am 11. Nukleotid der durch SEQ ID NR: 10 definierten Sequenz im BUB1b-Gen handelt es sich um T/T.

3. Verfahren nach Anspruch 1, wobei das Risiko des Auftretens von Granulozytopänie in dem Falle, in welchem es sich bei dem vom Patienten isolierten Gen um einen oder mehrere der folgenden Punkte (F) bis (J) handelt, gering ist:
(F) bei dem Genotyp am 11. Nukleotid der durch SEQ ID NR: 6 definierten Sequenz im BUB1b-Gen handelt es sich um A/G oder G/G;
(G) bei dem Genotyp am 11. Nukleotid der durch SEQ ID NR: 7 definierten Sequenz im BUB1b-Gen handelt es sich um G/T oder G/G;
(H) bei dem Genotyp am 11. Nukleotid der durch SEQ ID NR: 8 definierten Sequenz im BUB1b-Gen handelt es sich um C/T oder T/T;
(I) bei dem Genotyp am 11. Nukleotid der durch SEQ ID NR: 9 definierten Sequenz im BUB1b-Gen handelt es sich um C/T oder T/T; und
(J) bei dem Genotyp am 11. Nukleotid der durch SEQ ID NR: 10 definierten Sequenz im BUB1b-Gen handelt es sich um C/T oder C/C.

4. Verfahren nach Anspruch 1, umfassend das Identifizieren eines genetischen Polymorphismus am 11. Nukleotid der durch SEQ ID NR: 4 definierten Sequenz im CYP2C8-Gen und eines genetischen Polymorphismus am 11. Nukleotid der durch SEQ ID NR: 6 definierten Sequenz im BUB1b-Gen in einem von dem Patienten isolierten Gen.

5. Verfahren nach Anspruch 4, wobei das Risiko des Auftretens von Granulozytopänie als hoch vorhergesagt wird, wenn es sich bei dem Genotyp am 11. Nukleotid der durch SEQ ID NR: 4 definierten Sequenz im CYP2C8-Gen um T/T handelt und bei dem Genotyp am 11. Nukleotid der durch SEQ ID NR: 6 definierten Sequenz im BUB1b-Gen um A/A oder G/G handelt.

6. Verfahren nach Anspruch 4, wobei das Risiko des Auftretens von Granulozytopänie als gering vorhergesagt wird, wenn es sich bei dem Genotyp am 11. Nukleotid der durch SEQ ID NR: 4 definierten Sequenz im CYP2C8-Gen um A/T oder A/A handelt und bei dem Genotyp am 11. Nukleotid der durch SEQ ID NR: 6 definierten Sequenz im BUB1b-Gen um A/G handelt.

7. Diagnosekit zum Vorhersagen des Risikos des Auftretens von durch eine Paclitaxeltherapie verursachter Granulozytopänie bei einem Patienten, umfassend ein Reagens zum Identifizieren eines genetischen Polymorphismus bzw. mehrerer genetischer Polymorphismen, ausgewählt aus der Gruppe, bestehend aus
einem genetischen Polymorphismus am 11. Nukleotid der durch SEQ ID NR: 6 definierten Sequenz im BUB1b-Gen,
einem genetischen Polymorphismus am 11. Nukleotid der durch SEQ ID NR: 7 definierten Sequenz im BUB1b-Gen,
einem genetischen Polymorphismus am 11. Nukleotid der durch SEQ ID NR: 8 definierten Sequenz im BUB1b-Gen,
einem genetischen Polymorphismus am 11. Nukleotid der durch SEQ ID NR: 9 definierten Sequenz im BUB1b-Gen,
einem genetischen Polymorphismus am 11. Nukleotid der durch SEQ ID NR: 10 definierten Sequenz im BUB1b-Gen,
in einem von dem Patienten isolierten Gen.

8. Diagnosekit nach Anspruch 7, wobei es sich bei dem Reagens um eine oder mehrere Nukleinsäuremolekül(e) handelt, das/die ausgewählt ist/sind aus der Gruppe bestehend aus:
einem Nukleinsäuremolekül mit:
einer Sequenz von mindestens 10 zusammenhängenden Nukleotiden, enthaltend das 11. Nukleotid der durch SEQ ID NR: 6 definierten Sequenz im BUB1b-Gen, oder einer dazu komplementären Sequenz; oder
einer Sequenz von mindestens 10 zusammenhängenden Nukleotiden neben dem 11. Nukleotid der durch SEQ ID NR: 6 definierten Sequenz im BUB1b-Gen, oder einer dazu komplementären Sequenz;
einem Nukleinsäuremolekül mit:
einer Sequenz von mindestens 10 zusammenhängenden Nukleotiden, enthaltend das 11. Nukleotid der durch SEQ ID NR: 7 definierten Sequenz im BUB1b-Gen, oder einer dazu komplementären Sequenz; oder
einer Sequenz von mindestens 10 zusammenhängenden Nukleotiden neben dem 11. Nukleotid der durch SEQ ID NR: 7 definierten Sequenz im BUB1b-Gen, oder einer dazu komplementären Sequenz;
einem Nukleinsäuremolekül mit:
einer Sequenz von mindestens 10 zusammenhängenden Nukleotiden, enthaltend das 11. Nukleotid der durch SEQ ID NR: 8 definierten Sequenz im BUB1b-Gen, oder einer dazu komplementären Sequenz; oder
einer Sequenz von mindestens 10 zusammenhängenden Nukleotiden neben dem 11. Nukleotid der durch SEQ ID NR: 8 definierten Sequenz im BUB1b-Gen, oder einer dazu komplementären Sequenz;
einem Nukleinsäuremolekül mit:
einer Sequenz von mindestens 10 zusammenhängenden Nukleotiden, enthaltend das 11. Nukleotid der durch SEQ ID NR: 9 definierten Sequenz im BUB1b-Gen, oder einer dazu komplementären Sequenz; oder
einer Sequenz von mindestens 10 zusammenhängenden Nukleotiden neben dem 11. Nukleotid der durch SEQ ID NR: 9 definierten Sequenz im BUB1b-Gen, oder einer dazu komplementären Sequenz; und
einem Nukleinsäuremolekül mit:
einer Sequenz von mindestens 10 zusammenhängenden Nukleotiden, enthaltend das 11. Nukleotid der durch SEQ ID NR: 10 definierten Sequenz im BUB1b-Gen, oder einer dazu komplementären Sequenz; oder
einer Sequenz von mindestens 10 zusammenhängenden Nukleotiden neben dem 11. Nukleotid der durch SEQ ID NR: 10 definierten Sequenz im BUB1b-Gen, oder einer dazu komplementären Sequenz.

9. Diagnosekit nach Anspruch 7, wobei das Reagens umfasst:
ein Nukleinsäuremolekül mit:
einer Sequenz von mindestens 10 zusammenhängenden Nukleotiden, enthaltend das 11. Nukleotid der durch SEQ ID NR: 4 definierten Sequenz im CYP2C8-Gen, oder einer dazu komplementären Sequenz; oder
einer Sequenz von mindestens 10 zusammenhängenden Nukleotiden neben dem 11. Nukleotid der durch SEQ ID NR: 4 definierten Sequenz im CYP2C8-Gen, oder einer dazu komplementären Sequenz; und
ein Nukleinsäuremolekül mit:
einer Sequenz von mindestens 10 zusammenhängenden Nukleotiden, enthaltend das 11. Nukleotid der durch SEQ ID NR: 6 definierten Sequenz im BUB1b-Gen, oder einer dazu komplementären Sequenz; oder
einer Sequenz von mindestens 10 zusammenhängenden Nukleotiden neben dem 11. Nukleotid der durch SEQ ID NR: 6 definierten Sequenz im BUB1b-Gen, oder einer dazu komplementären Sequenz.

10. Diagnosekit nach Anspruch 7, wobei es sich bei dem Reagens um einen oder mehrere PCR-Primerpaare handelt, das/die ausgewählt ist/sind aus der Gruppe, bestehend aus:
einem PCR-Primerpaar, das dazu bestimmt ist, DNA zu amplifizieren, die einem Bereich entspricht, der das 11. Nukleotid der durch SEQ ID NR: 6 definierten Sequenz im BUB1b-Gen enthält;
einem PCR-Primerpaar, das dazu bestimmt ist, DNA zu amplifizieren, die einem Bereich entspricht, der das 11. Nukleotid der durch SEQ ID NR: 7 definierten Sequenz im BUB1b-Gen enthält;
einem PCR-Primerpaar, das dazu bestimmt ist, DNA zu amplifizieren, die einem Bereich entspricht, der das 11. Nukleotid der durch SEQ ID NR: 8 definierten Sequenz im BUB1b-Gen enthält;
einem PCR-Primerpaar, das dazu bestimmt ist, DNA zu amplifizieren, die einem Bereich entspricht, der das 11. Nukleotid der durch SEQ ID NR: 9 definierten Sequenz im BUB1b-Gen enthält;
einem PCR-Primerpaar, das dazu bestimmt ist, DNA zu amplifizieren, die einem Bereich entspricht, der das 11. Nukleotid der durch SEQ ID NR: 10 definierten Sequenz im BUB1b-Gen enthält.

11. Diagnosekit nach Anspruch 7, wobei das Reagens umfasst:
ein PCR-Primerpaar, das dazu bestimmt ist, DNA zu amplifizieren, die einem Bereich entspricht, der das 11. Nukleotid der durch SEQ ID NR: 4 definierten Sequenz im CYP2C8-Gen enthält; und
ein PCR-Primerpaar, das dazu bestimmt ist, DNA zu amplifizieren, die einem Bereich entspricht, der das 11. Nukleotid der durch SEQ ID NR: 6 definierten Sequenz im BUB1b-Gen enthält.

## Revendications

1. Procédé pour prédire le risque d'apparition d'une granulocytopénie provoquée par un traitement au paclitaxel chez un sujet, comprenant l'identification, dans un gène isolé du sujet, d'un ou plusieurs polymorphismes génétiques choisis dans le groupe consistant en :
un polymorphisme génétique sur le 11ème nucléotide de la séquence définie par SEQ ID N°6 dans le gène BUB1b,
un polymorphisme génétique sur le 11ème nucléotide de la séquence définie par SEQ ID N°7 dans le gène BUB1b,
un polymorphisme génétique sur le 11ème nucléotide de la séquence définie par SEQ ID N°8 dans le gène BUB1b,
un polymorphisme génétique sur le 11ème nucléotide de la séquence définie par SEQ ID N°9 dans le gène BUB1b,
un polymorphisme génétique sur le 11 ème nucléotide de la séquence définie par SEQ ID N°10 dans le gène BUB1b.

2. Procédé selon la revendication 1, dans lequel il est prévu que le risque d'apparition d'une granulocytopénie soit élevé dans le cas dans lequel le gène isolé du sujet est l'un ou plusieurs des génotypes (A) à (E) ci-après :
(A) le génotype sur le 11 ème nucléotide de la séquence définie par SEQ ID N°6 dans le gène BUB1b est A/A ;
(B) le génotype sur le 11 ème nucléotide de la séquence définie par SEQ ID N°7 dans le gène BUB1b est T/T ;
(C) le génotype sur le 11 ème nucléotide de la séquence définie par SEQ ID N°8 dans le gène BUB1b est C/C ;
(D) le génotype sur le 11 ème nucléotide de la séquence définie par SEQ ID N°9 dans le gène BUB1b est C/C ; et
(E) le génotype sur le 11 ème nucléotide de la séquence définie par SEQ ID N°10 dans le gène BUB1b est T/T.

3. Procédé selon la revendication 1, dans lequel il est prédit que le risque d'apparition d'une granulocytopénie soit faible dans le cas dans lequel le gène isolé du sujet est un ou plusieurs des génotypes (F) à (J) ci-après :
(F) le génotype sur le 11 ème nucléotide de la séquence définie par SEQ ID N°6 dans le gène BUB1b est A/G ou G/G ;
(G) le génotype sur le 11ème nucléotide de la séquence définie par SEQ ID N°7 dans le gène BUB1b est G/T ou G/G ;
(H) le génotype sur le 11 ème nucléotide de la séquence définie par SEQ ID N°8 dans le gène BUB1b est C/T ou T/T ;
(I) le génotype sur le 11 ème nucléotide de la séquence définie par SEQ ID N°9 dans le gène BUB1b est C/T ou T/T ; et
(J) le génotype sur le 11ème nucléotide de la séquence définie par SEQ ID N°10 dans le gène BUB1b est C/T ou C/C.

4. Procédé selon la revendication 1, comprenant l'identification, dans un gène isolé du sujet, d'un polymorphisme génétique sur le 11ème nucléotide de la séquence définie par SEQ ID N°4 dans le gène CYP2C8, et d'un polymorphisme génétique sur le 11 ème nucléotide de la séquence définie par SEQ ID N°6 dans le gène BUB1b.

5. Procédé selon la revendication 4, dans lequel il est prédit que le risque d'apparition d'une granulocytopénie soit élevé quand le génotype sur le 11 ème nucléotide de la séquence définie par SEQ ID N°4 dans le gène CYP2C8 est T/T, et que le génotype sur le 11ème nucléotide de la séquence définie par SEQ ID N°6 dans le gène BUB1b est A/A ou G/G.

6. Procédé selon la revendication 4, dans lequel il est prédit que le risque d'apparition d'une granulocytopénie soit faible quand le génotype sur le 11 ème nucléotide de la séquence définie par SEQ ID N°4 dans le gène CYP2C8 est A/T ou A/A, et que le génotype sur le 11ème nucléotide de la séquence définie par SEQ ID N°6 dans le gène BUB1b est A/G.

7. Trousse de diagnostic pour prédire le risque d'apparition d'une granulocytopénie provoquée par un traitement au paclitaxel chez un sujet, comprenant un réactif pour l'identification, dans un gène isolé du sujet, d'un ou plusieurs polymorphismes génétiques choisis dans le groupe consistant en :
un polymorphisme génétique sur le 11ème nucléotide de la séquence définie par SEQ ID N°6 dans le gène BUB1b,
un polymorphisme génétique sur le 11ème nucléotide de la séquence définie par SEQ ID N°7 dans le gène BUB1b,
un polymorphisme génétique sur le 11ème nucléotide de la séquence définie par SEQ ID N°8 dans le gène BUB1b,
un polymorphisme génétique sur le 11ème nucléotide de la séquence définie par SEQ ID N°9 dans le gène BUB1b,
un polymorphisme génétique sur le 11ème nucléotide de la séquence définie par SEQ ID N°10 dans le gène BUB1b.

8. Trousse de diagnostic selon la revendication 7, dans laquelle le réactif est constitué d'une ou plusieurs molécules d'acide nucléique choisies dans le groupe consistant en :
une molécule d'acide nucléique ayant une séquence d'au moins 10 nucléotides contigus contenant le 11ème nucléotide de la séquence définie par SEQ ID N°6 dans le gène BUB1b, ou une séquence qui lui est complémentaire ; ou une séquence d'au moins 10 nucléotides contigus, adjacente au 11ème nucléotide de la séquence définie par SEQ ID N°6 dans le gène BUB1b, ou une séquence qui lui est complémentaire ;
une molécule d'acide nucléique ayant une séquence d'au moins 10 nucléotides contigus contenant le 11ème nucléotide de la séquence définie par SEQ ID N°7 dans le gène BUB1b, ou une séquence qui lui est complémentaire ; ou une séquence d'au moins 10 nucléotides contigus, adjacente au 11ème nucléotide de la séquence définie par SEQ ID N°7 dans le gène BUB1b, ou une séquence qui lui est complémentaire ;
une molécule d'acide nucléique ayant une séquence d'au moins 10 nucléotides contigus contenant le 11ème nucléotide de la séquence définie par SEQ ID N°8 dans le gène BUB1b, ou une séquence qui lui est complémentaire ; ou une séquence d'au moins 10 nucléotides contigus, adjacente au 11ème nucléotide de la séquence définie par SEQ ID N°8 dans le gène BUB1b, ou une séquence qui lui est complémentaire ;
une molécule d'acide nucléique ayant une séquence d'au moins 10 nucléotides contigus contenant le 11ème nucléotide de la séquence définie par SEQ ID N°9 dans le gène BUB1b, ou une séquence qui lui est complémentaire ; ou une séquence d'au moins 10 nucléotides contigus, adjacente au 11ème nucléotide de la séquence définie par SEQ ID N°9 dans le gène BUB1b, ou une séquence qui lui est complémentaire ; et
une molécule d'acide nucléique ayant une séquence d'au moins 10 nucléotides contigus contenant le 11ème nucléotide de la séquence définie par SEQ ID N°10 dans le gène BUB1b, ou une séquence qui lui est complémentaire ; ou une séquence d'au moins 10 nucléotides contigus, adjacente au 11ème nucléotide de la séquence définie par SEQ ID N°10 dans le gène BUB1b, ou une séquence qui lui est complémentaire.

9. Trousse de diagnostic selon la revendication 7, dans laquelle le réactif comprend :
une molécule d'acide nucléique ayant une séquence d'au moins 10 nucléotides contigus contenant le 11ème nucléotide de la séquence définie par SEQ ID N°4 dans le gène CYP2C8, ou une séquence qui lui est complémentaire, ou une séquence d'au moins 10 nucléotides contigus, adjacente au 11ème nucléotide de la séquence définie par SEQ ID N°4 dans le gène CYP2C8, ou une séquence qui lui est complémentaire ; et
une molécule d'acide nucléique ayant une séquence d'au moins 10 nucléotides contigus contenant le 11ème nucléotide de la séquence définie par SEQ ID N°6 dans le gène BUB1b, ou une séquence qui lui est complémentaire ; ou une séquence d'au moins 10 nucléotides contigus, adjacente au 11ème nucléotide de la séquence définie par SEQ ID N°6 dans le gène BUB 1 b, ou une séquence qui lui est complémentaire.

10. Trousse de diagnostic selon la revendication 7, dans laquelle le réactif est constitué d'une ou plusieurs paires d'amorces PCR choisies dans le groupe consistant en :
une paire d'amorces PCR conçue de façon à amplifier l'ADN correspondant à la région contenant le 11ème nucléotide de la séquence définie par SEQ ID N°6 dans le gène BUB1b ;
une paire d'amorces PCR conçue de façon à amplifier l'ADN correspondant à la région contenant le 11ème nucléotide de la séquence définie par SEQ ID N°7 dans le gène BUB1b ;
une paire d'amorces PCR conçue de façon à amplifier l'ADN correspondant à la région contenant le 11ème nucléotide de la séquence définie par SEQ ID N°8 dans le gène BUB1b ;
une paire d'amorces PCR conçue de façon à amplifier l'ADN correspondant à la région contenant le 11ème nucléotide de la séquence définie par SEQ ID N°9 dans le gène BUB1b ;
une paire d'amorces PCR conçue de façon à amplifier l'ADN correspondant à la région contenant le 11ème nucléotide de la séquence définie par SEQ ID N°10 dans le gène BUB1b.

11. Trousse de diagnostic selon la revendication 7, dans lequel le réactif comprend :
une paire d'amorces PCR conçue de façon à amplifier l'ADN correspondant à la région contenant le 11ème nucléotide de la séquence définie par SEQ ID N°4 dans le gène CYP2C8 ; et
une paire d'amorces PCR conçue de façon à amplifier l'ADN correspondant à la région contenant le 11ème nucléotide de la séquence définie par SEQ ID N°6 dans le gène BUB1b.
